# EUROPEAN PATENT APPLICATION

(11) **EP 2 082 693 A1**
(43) Date of publication of application: **29.07.2009**
(21) Application number: 09000810.3
(22) Date of filing: 21.01.2009
(51) Int. Cl.: A61B 17/12, A61M 25/06, A61M 25/10, A61B 17/34

(54) **Blood flow blocking catheter**

(30) Priority: 28.01.2008 JP 2008016008
(71) Applicant: Asahikawa Medical College, Asahikawa-shi Hokkaido 078-8510 (JP); Nipro Corporation, Kita-ku, Osaka 531-8510 (JP)
(72) Inventor: Sasajima, Tadahiro, Midorigaoka-Higashi Asahikawa-shi Hokkaido 078-8510 (JP); Miyagawa, Katsuya, Osaka-shi Osaka 531-8510 (JP)
(74) Representative: Hager, Thomas Johannes

(57) **Abstract**

A blood flow blocking catheter, comprises: a puncture needle component having a puncture needle capable of puncturing a vein; and a sheath that covers said puncture needle component, and a balloon catheter that can be inserted into and removed from a lumen of the puncture needle component and has at its distal end a balloon that can block off blood flow. With the present invention, a balloon catheter can be easily inserted into a vein, and the balloon inflated, without making a large incision in the vein. Therefore, the temporary obstruction of blood flow with a balloon catheter can be carried out more easily, and this simplifies the surgery and makes it less invasive. Thus, it is possible to obtain a blood flow blocking catheter with which vascular anastomosis can be performed more safely and in less time.

## Description

### BACKGROUND OF THE INVENTION

### 1. Field of the Invention

The present invention relates to a blood flow blocking catheter that is used to temporarily block off blood flow during vascular surgery.

### 2. Background Information

Medical procedures that have been widely performed in the past include, for example, to dialyze blood by circulating the blood outside the body, to collect and examine blood, or to inject drugs or nutrients into a vein, all by means of a catheter that is inserted into a vein and left in place.

Catheters come in many different shapes and structures as dictated by the procedure involved, and include catheters with a single-tube structure, as well as catheters with a composite structure comprising two or more tubes.

Furthermore, vascular anastomosis, in which the blood flow is temporarily blocked off with forceps, is performed in vascular surgery, but there are often pathophysiologies in which blocking off blood flow with forceps is exceedingly difficult, such as in cases in which there is a high degree of calcification of the arterial walls, or cases in which there are high adhesions between arterial walls and surrounding tissue in revision surgery. With cases such as these, blood flow is often blocked off with a balloon catheter in order to simply such surgery and reduce its invasiveness, but inserting the catheter requires an arteriotomy to be performed, and with the aorta or a median artery, the insertion itself usually causes distress, so there has been a need for the development of a device that is easier to insert.

At present, as a clinical procedure in which blood flow is blocked off by inserting a balloon catheter through an incision and inflating the balloon, a blood flow stopping catheter has already been disclosed in which an incision is made in part of an artery during arteriolar anastomosis, and particularly in coronary bypass surgery, a balloon catheter is inserted, and the balloon is inflated to stop blood flow at the center and on both sides of the periphery of the incision (see Japanese Laid-Open Patent Application H10-127772, for example).

However, while a method in which an incision is made in part of an artery in order to insert a balloon catheter into a vein is possible with small arteries such as the coronary arteries, with the aorta and median arteries, the blocking balloon has to be large, and when the balloon catheter is inserted, a correspondingly large incision has to be made, which results in a large amount of hemorrhaging from the incision and the attendant difficulty in insertion, so this does not necessarily make the surgery simpler or less invasive. Because of these problems, with large veins, the veins have to be sutured after the catheter has been removed, and this not only means that the surgery takes longer, but also increases the burden on the patient.

Thus, a method in which an incision is made in an artery and a balloon catheter is inserted therein entails problems both during balloon catheter insertion and after the removal of the balloon catheter. There has been a need for the development of a blood flow blocking catheter that would allow a balloon catheter to be easily inserted and easily removed.

### SUMMARY OF THE INVENTION

It is an object of the present invention to provide a blood flow blocking catheter with which a balloon catheter can be easily inserted into a vein without making an incision in the vein, and blood flow temporarily blocked off.

The present invention provides a blood flow blocking catheter, comprising:
a puncture needle component having a puncture needle capable of puncturing a vein; and
a sheath that covers said puncture needle component, and
a balloon catheter that can be inserted into and removed from a lumen of the puncture needle component and has at its distal end a balloon that can block off blood flow.

With the present invention, a balloon catheter can be easily inserted into a vein, without making a large incision in the vein and the balloon inflated. Therefore, the temporary obstruction of blood flow with a balloon catheter can be carried out more easily, and this simplifies the surgery and makes it less invasive. Thus, it is possible to obtain a blood flow blocking catheter with which vascular anastomosis can be performed more safely and in less time.

### BRIEF DESCRIPTION OF THE DRAWINGS

FIG. 1 is an overall plan view of the blood flow blocking catheter pertaining to the present invention.
FIG. 2 is a plan view illustrating an example of a balloon catheter, which is one of the constituent parts of the present invention.
FIG. 3 is a plan view of the state when the balloon of the balloon catheter has been inflated.
FIG. 4 is a plan view of another example of the balloon catheter.
FIGS. 5 to 8 are diagrams illustrating how blood flow is blocked using the blood flow blocking catheter pertaining to the present invention.
FIG. 9 shows another embodiment of a blood flow blocking catheter, in which (a) is an overall plan view, (b) is a detail enlargement of the inner core in this embodiment, and (c) illustrates how the blood flow blocking catheter of this embodiment is used to block off blood flow.
FIG. 10 shows the relationship between the inner core and the size of the puncture needle, in which (a) is a diagram of a puncture needle with a small diameter that that does not have an inner core, (b) is a diagram of a puncture needle with a large diameter that does have an inner core, and (c) is a simplified diagram of the inner core.

### DETAILED DESCRIPTION OF THE PREFERRED EMBODIMENTS

An embodiment of the blood flow blocking catheter pertaining to the present invention will now be described in detail through reference to the drawings.

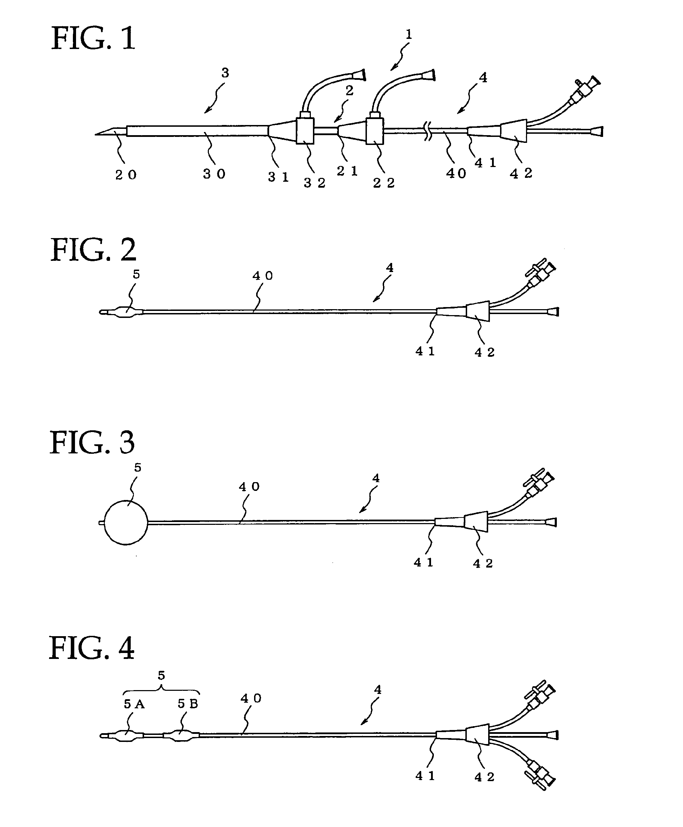

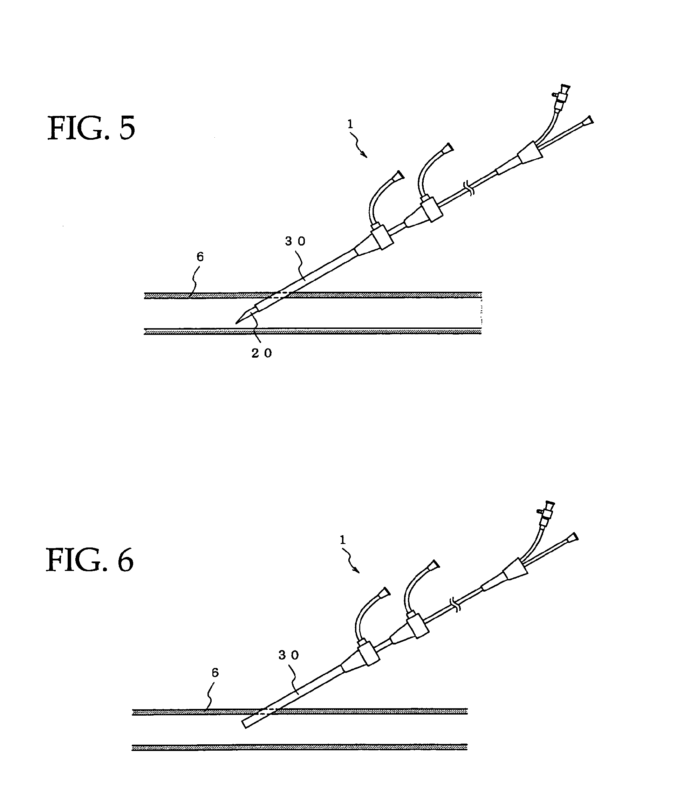

FIG. 1 is an overall plan view of the blood flow blocking catheter pertaining to the present invention. FIG. 2 is a plan view illustrating an example of a balloon catheter, which is one of the constituent parts of the present invention. FIG. 3 is a plan view of the state when the balloon of the balloon catheter has been inflated. FIG. 4 is a plan view of another example of the balloon catheter. FIGS. 5 to 8 are diagrams illustrating how blood flow is blocked using the blood flow blocking catheter pertaining to the present invention.

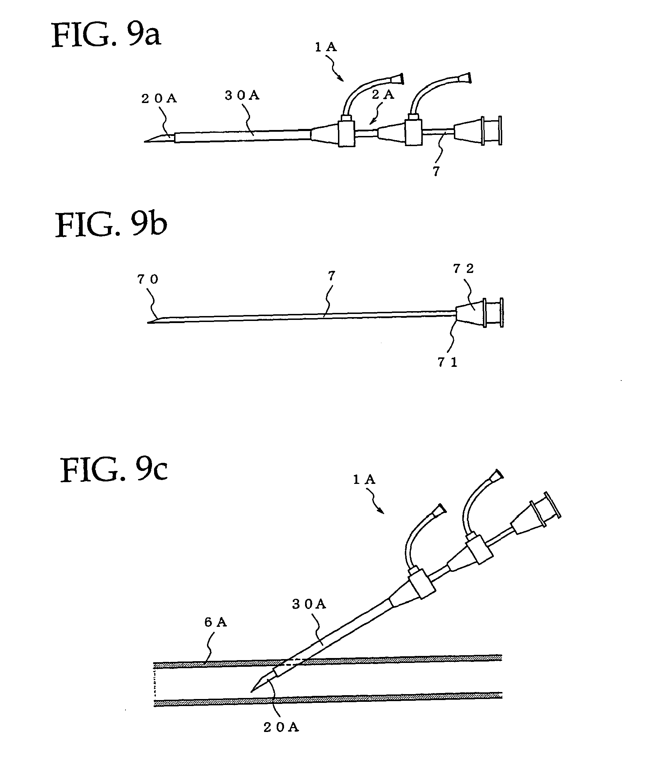

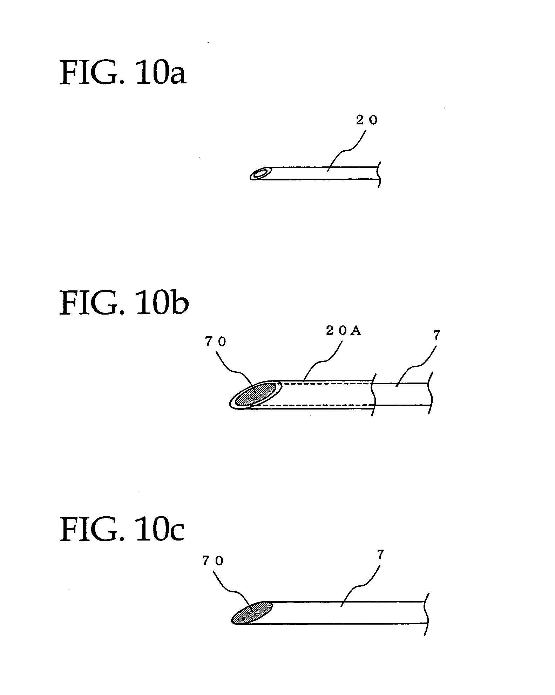

FIG. 9 shows another embodiment of a blood flow blocking catheter, in which (a) is an overall plan view, (b) is a detail enlargement of the inner core in this embodiment, and (c) illustrates how the blood flow blocking catheter of this embodiment is used to block off blood flow. FIG. 10 shows the relationship between the inner core and the size of the puncture needle, in which (a) is a diagram of a puncture needle with a small diameter that that does not have an inner core, (b) is a diagram of a puncture needle with a large diameter that does have an inner core, and (c) is a simplified diagram of the inner core.

As shown in FIGS. 1 to 4, the blood flow blocking catheter 1 of the present invention comprises a puncture needle component 2, a sheath component 3, and a balloon catheter 4. The puncture needle component 2 has a puncture needle 20 and a nonreturn valve connector 22 that is attached to the proximal end (base end) 21 of this puncture needle. The sheath component 3 has a sheath 30 and a nonreturn valve connector 32 that is attached to the proximal end 31 of this sheath. The balloon catheter 4 has a shaft 40, a balloon 5 that is attached to the distal end of this shaft, and a connector 42 and a two-way stopcock that are attached to the proximal end 41 of this shaft. The puncture needle 20 of the puncture needle component 2 can be inserted into the sheath 30, and the balloon 5 and the shaft 40 of the balloon catheter 4 can be inserted into the lumen of the puncture needle 20.

The puncture needle 20 is hard enough that it can puncture a vein, and its distal end has a tapered shape that is cut at an acute angle. The cross section is circular or has a shape that is close to circular, and is preferably about 1.9 to about 5.9 mm in outside diameter. Examples of the material from which the puncture needle 20 is formed include stainless steel, brass, and other metal materials, polyolefin, polyamide, polyester, fluorocarbon resin, silicone resin, polyvinyl chloride, polyurethane, and other plastics, natural rubber, and blade tubes that are a combination of plastic and stainless steel.

The sheath 30 is soft enough that it will not damage a vein, and its cross section is circular or has a shape that is close to circular, and is preferably about 2 to about 6 mm in outside diameter. Examples of material from which the sheath 30 is formed include polyolefin, polyamide, polyester, fluorocarbon resin, silicone resin, polyvinyl chloride, polyurethane, and other plastics, natural rubber, stainless steel, and blade tubes that are a combination of plastic and stainless steel.

The shaft 40 of the balloon catheter 4 can be inserted into and removed from the puncture needle component 2, and its distal end is equipped with the balloon 5, which is able to block off blood flow. Therefore, this can be inserted into a vein via the sheath 30 and the puncture needle 20. The shaft 40 is soft enough that it will not damage a vein, and its cross section is circular or has a shape that is close to circular, and is preferably about 1.5 to about 5.5 mm in outside diameter. The shaft 40 is provided with a lumen (not shown) for injecting physiological saline to inflate the balloon 5, and the number of lumens will vary with the intended application. The shaft 40 may be a double lumen or triple lumen provided with a guide wire or a liquid infusion lumen (not shown), and may be a two-wall or three-wall tube. Examples of materials that can be used to form the shaft include polyolefin, polyamide, polyester, fluorocarbon resin, silicone resin, polyvinyl chloride, polyurethane, and other plastics, natural rubber, stainless steel, and blade tubes that are a combination of plastic and stainless steel.

The balloon 5 can be inflated (see FIG. 3) to a size that allows blood flow to be blocked off (a vein to be obstructed), and the maximum vein diameter in which it can be used is about 40 mm, so its outside diameter is preferably about 2 to about 6 mm, and its elongation when inflated is preferably about 5 to about 8 times. As shown in the example in FIG. 4, two of the balloons 5 (5A and 5B) may be provided ahead of time to prevent the leakage of blood if a balloon rupture. Alternatively, three or more balloons may be provided. Thus, providing two or more balloons allows blood flow to be blocked off with one balloon, even thorough other balloon ruptures, so it can be the safety blood flow blocking catheter. The material used to form the balloon 5 can be any one that can expand and contract, such as natural rubber or a synthetic rubber such as silicone rubber or polyisoprene.

The balloon 5 can be attached to the shaft 40 by using a cyanoacrylate- or silicone-based adhesive, for example to bond the two ends of the balloon 5. In the insertion of the balloon 5 into the puncture needle, the balloon is preferably put under a suitable state of negative pressure first.

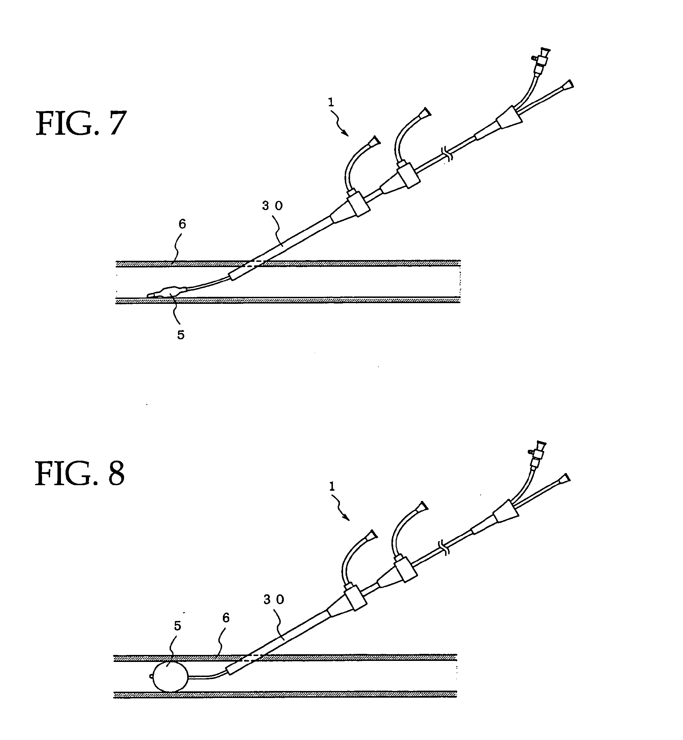

Next, how blood flow is blocked off using the blood flow blocking catheter of the present invention will be described through reference to the drawings (FIGS. 5 to 8).
First, the blood flow blocking catheter 1 shown in FIG. 1 is readied, the distal end of the puncture needle 20 is left protruding from the sheath 30, and a vein 6 is punctured (see FIG. 5). After it has been confirmed that the sheath tip has been inserted into the vein, the puncture needle 20 is pulled back (see FIG. 6). The distal end of the balloon catheter 4 is then inserted into the vein to dispose the balloon 5 at the required position in the vein (see FIG. 7). Physiological saline containing an imaging agent, for example, is then injected into the balloon 5, and the balloon 5 is inflated until it obstructs the interior of the vein and blocks off blood flow (see FIG. 8). A bypass may be provided separately if it will be necessary to allow the blood to flow. After this, although not depicted in the drawings, vascular anastomosis of the surgical site is performed while blood flow is blocked off, the catheter is removed to restore blood flow, and the operation is concluded.

If the puncture needle 20 here is small in diameter, then sticking the sharp distal end that has been cut to a taper into the vein will merely form a slit, and will not make a perforation hole in the vein. If the puncture needle 20 has a large diameter, however, there is the risk that the hole at the distal end will make a perforation of the vein. Therefore, an inner core is installed that has a cut face with an angle that matches the distal end shape of the puncture needle and that can be inserted into and removed from the lumen of the puncture needle component, so that the puncture needle tip will be solid, and even if the large-diameter puncture needle 20 is stuck into the vein, it can be insert and removed without making a perforation hole in the vein.

For example, as shown in FIG. 9a, by using a blood flow blocking catheter 1A in which a solid inner core 7 is installed inside a large-diameter sheath 30A, sticking a large-diameter puncture needle 20A into the vein will not make a perforation hole in the vein.

The distal end of the inner core 7 shown in FIG. 9b has a cut face 70 whose angle matches the distal end shape of the puncture needle 20A. Also, a nonreturn valve connector 72 is provided to the proximal end 71 of the inner core 7. Therefore, when the inner core 7 is inserted into the large-diameter sheath 30A and the cut face 70 is matched up with the tapered distal end of the puncture needle 20A, a sharp, solid needle tip is formed. When the blood flow blocking catheter 1A in this state is stuck into the vein 6A as shown in FIG. 9c, it merely forms a semicircular wound in the vein surface, and does not leave a perforation hole.

Therefore, after the large-diameter sheath 30A in which the inner core 7 has been installed is inserted into the vein, the inner core 7 is removed, the above-mentioned balloon catheter is inserted into the vein, and the required procedure has been completed, when the blood flow blocking catheter 1A is pulled out of the vein 6A, the wound in the vein surface will close up quickly, and the vein will recover in a short time.

The inner core 7 may be made of plastic or metal, just as with the sheath, with no particular restrictions on the material, as long as it will slide smoothly through the sheath.

Thus, even with the thick puncture needle 20A, when the puncture, needle 20A is stuck into the vein when this inner core 7 has been installed, the puncture needle can be inserted and removed without leaving an open hole in the vein.

The small-diameter puncture needle 20 shown in FIG. 10a will not form a round hole in the vein surface even through no inner core is installed to plug up the hole in the needle. However, with the large-diameter puncture needle 20A shown in FIG. 10b, there is the risk that a round hole will be leave in the punctured surface of the vein. Therefore, the inner core 7 having the cut face 70 shown in FIG. 10c is installed in the puncture needle 20A to prevent a perforation hole from being left when the needle tip is stuck into the vein.

Naturally, an inner core of a specific size may be installed in the lumen of the small-diameter puncture needle 20, and an inner core having a cut face with an angle that matches the distal end shape of the puncture needle and that can be inserted into and removed from the lumen of the puncture needle may be used.

Also, since the nonreturn valve that prevents the leakage of blood is used for the proximal end of the puncture needle component, for the proximal end of the sheath, and for the proximal end of the inner core, the result is a blood flow blocking catheter with which no blood leaks to the outside when the puncture needle is stuck into the vein.

With the present invention, the balloon catheter can be easily inserted into a vein, even thorough a vein is a large artery, without making a large incision in the vein. The balloon can be inflated, therefore, the temporary obstruction of blood flow with a balloon catheter can be carried out more easily, and this simplifies the surgery and makes it less invasive. Thus, it is possible to obtain a blood flow blocking catheter with which vascular anastomosis can be performed more safely and in less time.

This application claims priority to Japanese Patent Application No. 2008-016008. The entire disclosure of Japanese Patent Application No. 2008-016008 is hereby incorporated herein by reference.
While only selected embodiments have been chosen to illustrate the present invention, it will be apparent to those skilled in the art from this disclosure that various changes and modifications can be made herein without departing from the scope of the invention as defined in the appended claims. Furthermore, the foregoing descriptions of the embodiments according to the present invention are provided for illustration only, and not for the purpose of limiting the invention as defined by the appended claims and their equivalents. Thus, the scope of the invention is not limited to the disclosed embodiments.

## Claims

1. A blood flow blocking catheter (1), comprising:
a puncture needle component (2) having a puncture needle (20) capable of puncturing a vein; and
a sheath component (3)that covers said puncture needle component (2), and
a balloon catheter (4) that can be inserted into and removed from a lumen of the puncture needle component (2) and has at its distal end a balloon (5) that can block off blood flow.

2. The catheter according to Claim 1, wherein the balloon catheter (4) has a plurality of the balloons (5A, 5B) which can be inflated individually.

3. The catheter according to Claim 1, comprising an inner core that can be inserted into and removed from the lumen of the puncture needle component (2).

4. The catheter according to Claim 3, wherein the inner core (7) has a cut face with an angle that matches the distal end shape of the puncture needle.

5. The catheter according to Claim 1, wherein a nonreturn valve is provided to the proximal end of the puncture needle component (2).

6. The catheter according to Claim 1, wherein a nonreturn valve is provided to the proximal end of the sheath (30).
